# EUROPEAN PATENT APPLICATION

(11) **EP 3 944 254 A1**
(43) Date of publication of application: **26.01.2022**
(21) Application number: 20187042.5
(22) Date of filing: 21.07.2020
(51) Int. Cl.: G16H 40/60

(54) **SYSTEM FOR DISPLAYING AN AUGMENTED REALITY AND METHOD FOR GENERATING AN AUGMENTED REALITY**

(71) Applicant: Siemens Healthcare GmbH, 91052 Erlangen (DE)
(72) Inventor: Alzaga, Amilcar, 90453 Nürnberg (DE)

(57) **Abstract**

The current invention concerns a system for displaying an augmented reality, comprising:
- a medical device for acquiring image data,
- a first display device for displaying the image data,
- a first tracking element for locating the medical device,
- a second tracking element for locating the first and a second display device,
- the system configured to:
- receive a planning dataset,
- generate the augmented reality based on the planning dataset and a spatial positioning of the medical device, wherein the planning dataset is spatially arranged with respect to the first display device according to detected spatial positionings of the first and second display device,

- the second display device for displaying the augmented reality.

The invention further concerns a method for generating an augmented reality and a computer program product.

## Description

The current invention concerns a system for displaying an augmented reality, a method for generating an augmented reality and a computer program product.

For supporting a medical staff, in particular a physician, during an examination and/or treatment of a subject, in particular intra-procedurally, often times pre-procedural medical images of the subject are used for planning and/or displayed to the medical staff. Generally, a highly realistic visualization of such medical images can be achieved by displaying an augmented reality (AR). In augmented reality applications real objects, in particular the subject, can be overlaid with virtual data and observed by the medical staff. However, a realistic display of the augmented reality often requires a precise registration within the virtual data and/ or between the virtual data and the subject, which can be complicated by subject motion.

Alternatively, medical devices, in particular a catheter and/or endoscope and/or laparoscope, can be used by the medical staff to intra-procedurally acquire, in particular real-time, image data from a region under examination. Adversely, existing medical devices, in particular rigid and/or articulated endoscopes, frequently lack the ability of changing a field-of-view and/or the ability of being optically, in particular visually, tracked.

Furthermore, there are medical devices which can be configured to acquire three-dimensional (3D) image data from the region under examination, in particular via a stereoscopic lens. However, these medical devices often possess only a small stereoscopic baseline, wherein the depth perception can be significantly less than the perception of human eyes. A missing or only small depth cue information with regard to the intra-procedural image data can significantly complicate a precise actuation of the medical device by the medical staff.

Moreover, changes and/or modifications to the intra-procedural medical images often require a processing of a video signal originating from the medical device. Adversely, this processing of the video signal can result in a noticeable time lag between the acquisition of the intra-procedural image data and its display, which can hamper a precise actuation of the medical device during the examination and/or treatment, in particular minimally invasive surgery.

Hence, it is an object of the current invention to provide depth cue information and/or pre-processed information and/or planning information to support a user intra-procedurally.

This object is achieved by the subject-matter according to each independent claim. Advantageous embodiments are described in the dependent claims.

In a first aspect, the current invention concerns a system for displaying an augmented reality. The system comprises a first display device, a second display device, a first tracking element, a second tracking element and a medical device. The medical device is configured to acquire image data of a region under examination of a subject and provide the image data to the first display device. In addition, the first display device is configured to display a graphical representation of the image data. Further, the first tracking element is configured to detect a spatial positioning of the medical device. Moreover, the second tracking element is configured to detect spatial positionings of the first display device and the second display device. In addition, the system is configured to receive a planning dataset. The system is further configured to generate the augmented reality based on the planning dataset and the detected spatial positioning of the medical device, wherein the planning dataset is spatially arranged with respect to the first display device in accordance with the detected spatial positionings of the first and second display device. In addition, the second display device is configured to display a graphical representation of the augmented reality.

The medical device can comprise a diagnostic and/or surgical device which can be configured to acquire, in particular intra-procedural and/or real-time, image data of the region under examination of the subject. The subject can be a human patient and/or an animal patient and/or a phantom. In particular, the medical device may comprise a catheter and/or endoscope and/or laparoscope, which can be configured to be at least partially placed inside the region under examination of the subject. The region under examination of the subject may comprise an anatomical region and/or a hollow organ and/or body cavity of the subject. In particular, the medical device can comprise an optical and/or electromagnetic and/or ultrasound sensor, which can be configured to capture the image data of the region under examination. The medical device can be configured to passively and/or actively acquire the image data. In the first case, the medical device can be configured to capture the image data. In the second case, the medical device can further comprise a source, in particular an optical source and/or fiber and/or an electromagnetic source and/or an ultrasound transducer, which can be configured to emit light and/or electromagnetic and/or ultrasonic waves. The sensor and source can be spatially arranged next to each other. Alternatively, the sensor and source can be spatially arranged apart from each other, in particular the source or sensor can be placed adjacent to and/or outside the region under examination. The medical device may further comprise a medical device interface, which can be configured to provide the image data to the first display device.

The image data can comprise a two-dimensional (2D) and/or three-dimensional (3D) representation of at least part of the region under examination. In addition, the image data can be time-resolved. The image data may further comprise metadata, wherein the metadata can comprise an information regarding an acquisition geometry and/or operating parameter of the medical device.

The first display device can comprise a monitor and/or display, which can be configured to display the graphical representation of the image data. Advantageously, the first display device can be configured to display the graphical representation of the image data in real-time.

The first tracking element can comprise a sensor, exemplary an electromagnetic and/or optical and/or ultrasound sensor, which can be configured to detect a spatial positioning of the medical device. Further, the first tracking element can be configured to detect and/or track and/or monitor the spatial positioning, in particular a spatial position and/or orientation, of the medical device, in particular in a coordinate frame of the subject and/or a reference coordinate frame. Furthermore, the medical device can comprise a first marker element, which can be identified and spatially located by the first tracking element. The first marker element can be embodied as a predefined shape and/or contour and/or segment of the medical device, in particular a fiducial marker and/or a predefined segment of the medical device. Furthermore, the first marker element can be embodied as at least one marker object which can be attached to the medical device, in particular on a distal and/or proximal portion of the medical device. Exemplary, the first tracking element can comprise a camera, in particular a mono and/or stereo camera, which can be configured to detect and/or track and/or monitor the medical device, in particular the first marker element.

Moreover, the first tracking element can be at least partially integrated in and/or attached to the medical device. In addition, the first tracking element may comprise an acceleration and/or inertia and/or position sensor, in particular a gyroscope. When the first tracking element is at least partially integrated in and/or attached to the medical device, the spatial positioning of the medical device can be detected and/or tracked without the first marker element.

Analogously, the second tracking element can comprise a sensor, exemplary an electromagnetic and/or optical and/or ultrasound sensor, which can be configured to, in particular simultaneously, detect a spatial positioning of the first and second display device. Alternatively, the second tracking element can comprise a first and a second sensor, wherein the first sensor can be configured to detect the spatial positioning of the first display device and the second sensor can be configured to detect the spatial positioning of the second display device. In particular, the second tracking element can be configured to detect and/or track the spatial positioning, in particular a spatial position and/or orientation, of the first and second display device, in particular relative to each other and/or in a common coordinate frame. Furthermore, the second tracking element can be configured to detect the spatial positionings of the first and second display element in the same reference coordinate frame in which the spatial positioning of the medical device is detected.

The first display device can comprise a second marker element and/or the second display device can comprise a third marker element, wherein the second tracking element can be configured to identify and spatially locate the second and/or the third marker element. The second marker element can be embodied as a predefined shape and/or contour and/or segment of the first display device, in particular a fiducial marker. Furthermore, the second marker element can be embodied as at least one marker object which can be attached to the first display device. Analogously, the third marker element can be embodied as a predefined shape and/or contour and/or segment of the second display device, in particular a fiducial marker. Moreover, the third marker element can be embodied as at least one marker object which can be attached to the second display device. Furthermore, the second tracking element can comprise a camera, in particular a mono and/or stereo camera, which can be configured to detect and/or track the first and/or second display device, in particular the second and/or the third marker element.

Advantageously, the second tracking element can be configured to detect a relative and/or absolute spatial positioning between the first and second display device. By way of example, the first and/or the second display device can comprise a sensor, in particular an acceleration and/or inertia and/or position sensor, which can be configured to detect and provide the spatial positioning, in particular the spatial position and/or orientation, of the respective display device to the second tracking element. This can be particularly helpful, if an obstacle occurs in a line-of-sight between the second tracking element and the first and/or second display device.

The system can comprise a system interface, which can be configured to receive the planning dataset, in particular via a wired and/or wireless transmission from a medical imaging system and/or a processing unit. The system interface can be configured to collect and/or readout data from an electronically readable storage medium and/or to receive data from a memory unit, in particular a database. The medical imaging system can be configured to acquire the planning dataset of the subject, in particular pre-procedurally. The planning dataset can comprise a 2D and/or 3D representation of at least part of the region under examination of the subject. In addition, the planning dataset can be time-resolved. The planning dataset may further comprise pre-processed information, for example annotations and/or anatomical landmarks and/or geometrical landmarks and/or enhanced features, in particular depth cue information. In addition, the planning dataset can comprise planning information regarding a path for a medical object, in particular the medical device, and/or information regarding a treatment plan. Furthermore, the planning dataset can at least partially comprise a representation of the region under examination where the medical device is placed and/or which is represented in the image data. Hence, the image data and the planning dataset can each comprise a representation of at least a common part of the region under examination. The planning dataset may further comprise metadata, wherein the metadata can comprise an information regarding an acquisition geometry and/or operating parameter of the medical imaging system during the acquisition of the planning dataset. In particular, the metadata can comprise an information regarding a spatial reference and/or registration with regard to the subject coordinate frame.

The medical imaging system for acquiring and/or providing the planning dataset can be a medical X-ray system, in particular a C-arm X-ray system, and/or a computed tomography system (CT) and/or a magnetic resonance imaging system (MRI) and/or a positron emission tomography system (PET) and/or an ultrasound imaging system. Further, the medical imaging system can be configured to provide the planning dataset to the system, in particular via the system interface. The medical imaging system for acquiring and/or providing the planning dataset can be a same or a different imaging modality than the medical device.

The system can be further configured to generate the augmented reality based on the planning dataset and the detected spatial positioning of the medical device. Advantageously, the augmented reality can comprise a 2D and/or 3D graphical representation of the planning dataset. The system can be configured to generate the graphical representation of the planning dataset based on a characteristic of the medical device, in particular a type and/or operating parameter and/or an acquisition parameter, and/or the detected spatial positioning of the medical device. By way of example, the graphical representation of the planning dataset can comprise depth cue information. By way of example, the depth cue information can comprise a color shading and/or virtual shadows and/or virtual light effects and/or blurring and/or enhanced geometric features. In addition, the system can be configured to generate the graphical representation of the planning dataset such that the graphical representation of the planning dataset matches at least partially with a field-of-view of the medical device. For this purpose, the system can be configured to transform, in particular rotate and/or translate and/or scale and/or deform and/or truncate, the planning dataset based on the detected spatial positioning and/or the characteristic of the medical device.

Advantageously, the second display device can be portable and/or wearable by a user. In addition, the second display device can be configured to display the graphical representation of the augmented reality (abbreviated: AR). Further, the second display device can be configured to be at least partially transparent. Moreover, the second display device can be configured to be worn by the user at least partially within a field-of-view of the user. The second display device can be configured to display a stereoscopic view of the augmented reality, in particular the graphical representation of the augmented reality, to the user. Hereby, the user can perceive the depth cue information from the augmented reality, in particular the graphical representation of the planning dataset, which can support the user in real-time when actuating the medical device intra-procedurally.

The second display device can comprise a second display interface, which can be configured to receive the augmented reality, in particular via a wired or wireless transmission from the processing unit. The second display interface can be configured to collect and/or readout data from an electronically readable storage medium and/or to receive data from a memory unit, in particular a database.

Furthermore, the system can be configured to spatially arrange the planning dataset, in particular the graphical representation of the planning dataset, with respect to the first display device in accordance with the detected spatial positionings of the first and second display device. In particular, the system can be configured to spatially arrange the graphical representation of the augmented reality comprising the graphical representation of the planning dataset with respect to the graphical representation of the image data, which is displayed on the first display device. The system can be configured to determine a spatial relation and/or registration between the first display device, which is displaying the graphical representation of the image data, and the second display device, which is displaying the graphical representation of the augmented reality, based on the detected spatial positionings of the first and second display device. Advantageously, the augmented reality, which is displayed by the second display device, can be at least partially overlaid and/or integrated into the graphical representation of the image data, which is displayed by the second display device. Hereby, the graphical representation of the augmented reality can be immersed into the graphical representation of the image data. Advantageously, at least part of the graphical representation of the augmented reality, in particular an anatomical and/or geometrical feature, can be spatially and/or visually matched to the graphical representation of the image data on the first display device. The spatial and/or visual matching of the graphical representation of the augmented reality may comprise a rotation and/or translation and/or scaling and/or deformation and/or truncation of at least part of the graphical representation of the augmented reality based on the detected relative spatial positioning between the first and second display device. Furthermore, the matching can comprise an at least regional adaption of an opacity and/or coloring of the graphical representation of the augmented reality, in particular based on an image parameter of the image data and/or a display parameter of the first display device.

The proposed system can permit an enhancement of the image data, which is displayed on the first display device, via the graphical representation of the planning dataset in the augmented reality. Consequently, the augmented reality can provide additional depth cue information and/or pre-processed information and/or planning information in an overlay with the image data to the user. In particular, the display of the image data on the first display device can remain unaffected and/or unaltered and/or non-delayed by the display of the augmented reality on the second display device.

In an especially preferred embodiment of the proposed system, the system can be further configured to generate the augmented reality comprising a virtual representation of the medical device based on the detected spatial positioning of the medical device.

The system can be configured to generate the augmented reality comprising a 2D and/or 3D arrangement of the planning dataset and the virtual representation of the medical device. In particular, the virtual representation of the medical device can comprise a 2D and/or 3D model of the medical device, which can be generated, in particular simulated and/or derived, based on a user input and/or a characteristic and/or an operating parameter and/or an acquisition parameter of the medical device. By way of example, the virtual representation of the medical device can comprise a volume model, in particular a mesh model, and/or a shape model. Alternatively, the virtual representation of the medical device can be selected from a library comprising virtual representations of different medical devices, wherein the selection can be based on a user input and/or a characteristic of the medical device.

The virtual representation of the medical device can be embodied as a realistic and/or simplified, in particular abstracted, model of the medical device, wherein a spatial extent and/or shape of the model can at least partially match the spatial extent and/or shape of the medical device. Furthermore, the virtual representation of the medical device can comprise enhanced geometric features, for example a contour and/or area and/or a longitudinal direction, and/or operating features, for example a planned path and/or a graphical representation of a field-of-view, of the medical device which can improve a visual perception by the user.

In addition, the virtual representation of the medical device can be used to calibrate the field-of-view of the medical device with regard to a specific coordinate frame, in particular the coordinate frame of the subject and/or the reference coordinate frame. Advantageously, the system can be configured to calibrate and/or register the field-of-view of the medical device, in particular with respect to the augmented reality, by calibrating the field-of-view of the virtual representation of the medical device.

The system can be further configured to spatially arrange the virtual representation of the medical device with respect to the planning dataset based on, in particular according to, the detected spatial positioning, in particular the spatial position and/or spatial orientation, of the medical device. By way of example, this can be implemented by registering the detected spatial positioning of the medical device and the planning dataset in the subject coordinate frame. Advantageously, the augmented reality can depict the virtual representation of the medical device and the graphical representation of the planning dataset in a virtual spatial arrangement, which can be in accordance with the real spatial arrangement of the medical device with respect to the region under examination of the subject. The system can be configured to at least partially overlay and/or integrate the virtual representation of the medical device and the graphical representation of the planning dataset for generating the augmented reality.

Advantageously, the user can hereby visually perceive the current and/or planned spatial positioning of the medical device with respect to the planning dataset via the augmented reality.

In an especially preferred embodiment of the proposed system, the system can be further configured to monitor at least one of the detected spatial positionings. In addition, the system can be configured to adapt the augmented reality when a change in the at least one of the detected spatial positionings occurs.

The first and/or second tracking element can be configured to monitor the spatial positionings of the respective device by either continuously or recurrently detecting the spatial positionings, in particular according to a predefined time interval. The first and/or second tracking element can further be configured to detect and/or signal a change in the respectively detected, in particular monitored, spatial positionings by comparing the current spatial positioning with a previously detected spatial positioning of the respective device. The comparison may include computing a difference between the previously and currently detected spatial positioning and comparing the difference to a predefined threshold. The first and/or second tracking element can be configured to signal the change in the respectively detected spatial positionings when the difference exceeds the predefined threshold. For each of the detected spatial positionings a different predefined threshold can be specified, in particular via a user input.

Advantageously, the system can be configured to adapt the augmented reality, in particular the graphical representation of the planning dataset and/or the virtual representation of the medical device, when a change in the spatial positioning of the medical device is detected by the first tracking element. Hereby, the adapted augmented reality can depict the graphical representation of the planning dataset and/or the virtual representation of the medical device in the virtual spatial arrangement, which can hence remain in accordance with the real spatial arrangement of the medical device with respect to the region under examination of the subject. The adaption of the augmented reality may comprise a rigid and/or non-rigid transformation of the graphical representation of the augmented reality, in particular the graphical representation of the planning dataset and/or the virtual representation of the medical device. The rigid and/or non-rigid transformation of the graphical representation of the augmented reality may comprise a rotation and/or translation and/or scaling and/or deformation and/or truncation of at least part of the augmented reality.

Furthermore, the system can be configured to adapt the augmented reality when the second tracking element detects a change in the relative spatial positioning between the first and second display device. If the spatial positionings of the first and second display device are changing uniformly, an adaption of the augmented reality may not become necessary.

When a change in the relative spatial positioning between the first and second display device is detected by the second tracking element, the graphical representation of the augmented reality can be adapted such that it immerses into the graphical representation of the image data displayed by the first display device. The system can be configured to adapt at least part of the graphical representation of the augmented reality such that it spatially and/or visually matches with the graphical representation of the image data, in particular an anatomical and/or geometrical feature. The adaption of the at least part of the graphical representation of the augmented reality can comprise a spatial and/or visual matching of the graphical representation of the augmented reality. The spatial and/or visual matching of the at least part of the graphical representation of the augmented reality can comprise a rotation and/or translation and/or scaling and/or deformation and/or truncation of the at least part of the graphical representation of the augmented reality based on the detected change in the relative spatial positioning between the first and second display device.

By, in particular continuously, adapting the augmented reality when a change in at least one of the detected spatial positionings is detected, an immersive display of the augmented reality and the image data can be ensured. In particular, when the second display device is embodied as a head-mounted display device, the augmented reality can be adapted whenever the head of the user is moved, and thereby the second display device, with respect to the first display device.

In an especially preferred embodiment of the proposed system, the second tracking element can be mounted to the second display device in a predefined position. In particular, the second tracking element can be attached to and/or at least partially integrated into the second display device such that a relative positioning between the second display device and the second tracking element remains unchanged. Advantageously, the predefined position of the second tracking element with respect to the second display device can permit an inherent registration between the spatial positioning of the second display device and the second tracking element, even when the second display device is moved. Advantageously, the second tracking element can be mounted to the second display device in a stationary predefined position and/or orientation. Thereby, the second tracking element can be configured to detect the spatial positioning of the first display device with respect to the second display device, in particular the relative spatial positioning between the two display devices.

In an especially preferred embodiment of the proposed system, the second tracking element can be arranged spatially apart from the first and second display device.

By way of example, the second tracking element can be mounted to a wall and/or floor and/or ceiling of a room, in which room the first and second display device are located. Advantageously, the second tracking element can be stationary, in particular with respect to the room, whilst the first and/or the second display device can be mobile.

By arranging the second tracking element spatially apart from the first and second display device, a physical weight and/or power consumption of the first and second display device can advantageously be reduced. In addition, when arranged spatially apart, the second tracking element can be configured to detect spatial positionings of further second display devices, which can be configured likewise to the second display device and may be worn by further users within the same room.

In an especially preferred embodiment of the proposed system, the system can be further configured to segment at least part of the planning dataset and generate the augmented reality based on the segmented planning dataset.

The system can be configured to segment a predefined anatomical and/or geometrical structure and/or region-of-interest of the planning dataset, in particular based on a user input. The segmentation can be based on a comparison between image values of the planning dataset and a predefined image threshold. Alternatively, the segmentation can be based on a shape-based and/or contour-based and/or pattern-based and/or landmark identification algorithm. Thereby, the most relevant information from the planning dataset for the current application can advantageously be incorporated into the generation of the planning dataset.

In an especially preferred embodiment of the proposed system, the first tracking element can be embodied as a medical imaging system.

In particular, the first tracking element can be embodied as a medical X-ray system, in particular a C-arm X-ray system, and/or a computed tomography system (CT) and/or a magnetic resonance imaging system (MRI) and/or a positron emission tomography system (PET) and/or an ultrasound imaging system. The first tracking element can be configured to, in particular recurrently and/or continuously, acquire further medical image data of the region under examination and to detect the spatial positioning of the medical device based on the further medical image data. Furthermore, the first tracking element can be configured to identify and locate the medical device in the further medical image data, in particular based on the first marker element. Advantageously, the first tracking element can be configured to identify and spatially locate the first marker element, in particular a graphical representation of the first marker element, in the further medical image data. Further, the first marker element can exhibit a spatial structure and/or shape and/or marker structure and/or landmark, which enables a detection of the spatial positioning of the first marker element based on its graphical representation in the further medical image data.

When the first tracking element is embodied as a medical imaging system and the planning dataset was acquired by the medical imaging system, the detected spatial positioning of the medical device can inherently be registered to the planning dataset. This can be of particular advantage for the generation of the augmented reality based on the planning dataset and the detected spatial positioning of the medical device.

In an especially preferred embodiment of the proposed system, the medical imaging system can be configured to acquire a device dataset. In addition, the system can be further configured to segment the medical device in the device dataset and generate the virtual representation of the medical device based on the segmented device dataset.

Advantageously, the further medical image data can comprise the device dataset, wherein the device dataset can comprise a graphical representation of the medical device. Advantageously, the device dataset can depict the graphical representation of the medical device comprising a contour and/or shape of at least part of the medical device. The system can be configured to segment the medical device, in particular the graphical representation of the medical device, in the device dataset. The segmentation can be based on a comparison between image values of the device dataset and a further predefined image threshold. Alternatively, the segmentation can be based on a shape-based and/or contour-based and/or pattern-based and/or landmark-based identification algorithm. Alternatively or in addition, the system can be configured to receive a characteristic of the medical device, in particular a type and/or operating parameter and/or an acquisition parameter of the medical device, wherein the segmentation can at least partially be based on the characteristic of the medical device.

Furthermore, the system can be configured to generate the virtual representation of the medical device based on the segmented device dataset. By way of example, the virtual representation of the medical device comprising a 2D and/or 3D model of the medical device can be generated and/or derived from the segmented device dataset, in particular a contour and/or surface in the segmented device dataset.

This embodiment advantageously permits the generation of a realistic virtual representation of the medical device, which can be included into the augmented reality.

According to a further exemplary embodiment of the proposed system, the first and second tracking element can be embodied as a single sensor. The single sensor can be embodied as an electromagnetic and/or optical and/or ultrasound sensor, which can be configured to, in particular simultaneously, detect the spatial positionings of the medical device, the first display device and the second display device. In particular, the single sensor can be configured to detect and/or track and/or monitor the spatial position and/or orientation of the medical device, the first display device and the second display device in a common coordinate frame.

Advantageously, the single sensor can be arranged spatially apart from any of the devices whose spatial positionings are tracked. Alternatively, the single sensor can be mounted to the first or second display device, which permits an inherent registration between the detected spatial positionings and the display device the single sensor is mounted to.

In an especially preferred embodiment of the proposed system, the second display device can be at least partially embodied as a head-mounted display device which is wearable by a user.

In particular, the second display device can be configured as a pair of goggles, in particular data goggles, and/or a helmet, in particular a data helmet, and/or a screen and/or projector. When the second display is worn by a user, in particular at least partially on the user's head, the second display device will move uniformly with the user, in particular the user's head. Since the second display device's spatial positioning can be detected by the second tracking element, the augmented reality, in particular the graphical representation of the augmented reality, can be adapted to any changes of the user's positioning, in particular the user's head positioning. Advantageously, this permits a continued display of the stereoscopic view of the augmented reality, in particular the graphical representation of the augmented reality, to the user, wherein the graphical representation of the augmented reality is immersed into the image data, which image data is displayed by the first display device.

According to a further exemplary embodiment of the proposed system, the first and/or second tracking element can comprise an optical and/or electromagnetic sensor. The sensor can be configured to identify and/or capture and/or spatially locate the device to be detected, in particular the medical device and/or the first display device and/or the second display device. Advantageously, the first and/or second tracking element can each comprise one sensor or multiple sensors, in particular multiple sensors of the same or different kind, which can enable a more robust detection of the spatial positioning of the respective device, in particular in a medical and/or surgical environment.

By way of example, the optical sensor can comprise a camera, in particular a mono and/or stereo and/or a depth camera, which is configured to detect and/or track and/or monitor the spatial positioning of respective device. Further, the electromagnetic sensor can be configured to detect changes in an electromagnetic field and/or electromagnetic waves caused and/or altered and/or emitted by the respective device to be detected. Moreover, the ultrasound sensor can be configured to emit and/or receive ultrasound waves which can be emitted and/or deflected and/or altered by the respective device to be detected.

Furthermore, the respective device to be detected, in particular the medical device and/or the first display device and/or the second display device, can comprise a marker element, in particular the first marker element and/or the second marker element and/or the third marker element, wherein the optical and/or electromagnetic and/or ultrasound sensor can be configured to identify and/or differentiate and/or detect the respective marker element. This embodiment of the proposed system can advantageously enable a precise and reliable detection of the spatial positionings of the medical device and/or the first display device and/or second display device.

According to a further exemplary embodiment of the proposed system, the medical device can be embodied as an endoscope and/or a catheter and/or a laparoscope.

Advantageously, the medical device can be configured to be at least partially placed inside the region under examination of the subject. The medical device can be embodied as a longitudinal diagnostic and/or surgical instrument, which can comprise an at least partially flexible and/or rigid segment. In addition, the medical device, in particular the endoscope and/or catheter and/or laparoscope, can comprise a lens segment, in particular at a proximal portion of the medical device, which can be configured to acquire the image data. In particular, the lens segment can comprise a rod lens.

The medical device can further be configured to be placed and/or moved by a user and/or a robot, in particular a catheter robot. In addition, the medical device can be configured to provide an information about its, in particular current, characteristic, in particular a type and/or operating parameter and/or an acquisition parameter and/or shape and/or penetration depth with regard to the region under examination, to the processing unit of the system. Based thereon, the virtual representation of the medical device can advantageously be generated and/or arranged in the augmented reality with higher precision. In addition, the system can be configured to adapt the augmented reality comprising the graphical representation of the planning dataset based on the characteristic of the medical device, in particular an operating parameter and/or an acquisition parameter, such that the graphical representation of the planning dataset matches at least partially with a field-of-view of the medical device.

In an especially preferred embodiment of the proposed system, the medical device, in particular in the exemplary embodiment as an endoscope and/or a catheter and/or a laparoscope, can comprise an angled lens for acquiring the image data. In other words, the field-of-view of the medical device, in particular the field-of-view of the angled lens, can be at least partially oriented sideways from a longitudinal direction of the medical device. The angled lens can comprise an optical element, in particular an optical lens and/or an optical fibre, which can be configured to, in particular bi-directionally, carry light between the region under examination, in particular the field-of-view, and a, in particular optical, sensor of the medical device, wherein the sensor can be configured to acquire, in particular capture, the image data.

Advantageously, the system can be configured to generate the graphical representation of the planning dataset such that the graphical representation of the planning dataset matches at least partially with the field-of-view of the medical device, in particular with the field-of-view of the angled lens.

In cases, where a spatial section around and/or adjacent to a longitudinal path of the medical device is to be imaged by the medical device, an angled lens can enable the medical device to acquire the image data of the spatial section without the need of turning and/or bending the lens segment of the medical device.

In a second aspect, the current invention concerns a, in particular computer-implemented, method for generating an augmented reality. In a first step a), a planning dataset is received. In a second step b), image data of a region under examination of a subject is received from a medical device. In a third step c), a graphical representation of the image data is displayed with a first display device. In a fourth step d), a spatial positioning of the medical device is detected with a first tracking element. In a fifth step e), spatial positionings of the first display device and the second display device are detected with a second tracking element. In a sixth step f), the augmented reality is generated based on the planning dataset and the detected spatial positioning of the medical device. Furthermore, the planning dataset is spatially arranged with respect to the first display device in accordance with the detected spatial positionings of the first and second display device. In a seventh step g), a graphical representation of the augmented reality is displayed with the second display device.

All remarks and advantages laid out above regarding the system for displaying an augmented reality also apply to the method for generating an augmented reality according to the invention and vice versa. Additional steps or sub-steps may be added regarding additional units according to the described embodiments of the system according to the invention, which can also be transferred to advantageous embodiments of the method for generating an augmented reality according to the invention and vice versa.

In an especially preferred embodiment of the proposed method, the augmented reality can be generated comprising a virtual representation of the medical device based on the detected spatial positioning of the medical device.

The augmented reality can be generated comprising a 2D and/or 3D arrangement of the planning dataset and the virtual representation of the medical device. Furthermore, the virtual representation of the medical device can comprise a 2D and/or 3D model of the medical device, which can be generated, in particular simulated and/or derived, based on a user input and/or a characteristic and/or an operating parameter and/or an acquisition parameter of the medical device. By way of example, the virtual representation of the medical device can comprise a volume model, in particular a mesh model, and/or a shape model. Alternatively, the virtual representation of the medical device can be selected from a library comprising virtual representations of different medical devices, wherein the selection can be based on a user input and/or a characteristic of the medical device.

The virtual representation of the medical device can be embodied as a realistic and/or simplified, in particular abstracted, model of the medical device, wherein a spatial extent and/or shape of the model can at least partially match the spatial extent and/or shape of the medical device. Furthermore, the virtual representation of the medical device can comprise enhanced geometric features, for example a contour and/or area and/or a longitudinal direction, and/or operating features, for example a planned path and/or a graphical representation of a field-of-view, of the medical device which can improve a visual perception by the user.

The virtual representation of the medical device can be spatially arranged with respect to the planning dataset based on the detected spatial positioning, in particular the spatial position and/or spatial orientation, of the medical device. By way of example, this can be implemented by registering the detected spatial positioning of the medical device and the planning dataset in the subject coordinate frame. Advantageously, the augmented reality can depict the virtual representation of the medical device and the graphical representation of the planning dataset in a virtual spatial arrangement, which is in accordance with the real spatial arrangement of the medical device with respect to the region under examination of the subject. The virtual representation of the medical device can be at least partially overlaid and/or integrated with the graphical representation of the planning dataset for generating the augmented reality.

Advantageously, the user can hereby visually perceive the current and/or planned spatial positioning of the medical device with respect to the planning dataset via the augmented reality.

In an especially preferred embodiment of the proposed method, the execution of steps b) to f) can be repeated when a change in the spatial positioning of the medical device is detected.

A change in the spatial positioning of the medical device can advantageously be detected by the first tracking element. In particular, the first tracking element can be configured to monitor the spatial positioning of the medical device by either continuously or recurrently detecting the spatial positioning, in particular according to a predefined time interval. Further, a difference between the previously and currently detected spatial positioning of the medical device can be computed and compared to a predefined threshold. By way of example, the predefined threshold can be determined via a user input. When the computed difference between the previously and currently detected spatial positioning of the medical device exceeds the predefined threshold, the execution of steps b) to f) can be repeated. By repeating steps b) to f) when a change in the spatial positioning of the medical device is detected, the augmented reality can be generated, in particular adapted, such that it advantageously depicts the graphical representation of the planning dataset and/or the virtual representation of the medical device in the virtual spatial arrangement in accordance with the real spatial arrangement of the medical device with respect to the region under examination of the subject. Hence, an immersive display of the augmented reality and the image data can be ensured.

In an especially preferred embodiment of the proposed method, the execution of steps e) to f) can be repeated when a change in the spatial positioning of the first and/or the second display device is detected.

A change in the spatial positioning of the first and/or the second display device can advantageously be detected by the second tracking element. In particular, the second tracking element can be configured to monitor the spatial positionings of the first and the second display device by either continuously or recurrently detecting the spatial positionings, in particular according to a predefined time interval. Further, a difference between the previously and currently detected spatial positioning of the first and/or the second display device can be computed and compared to a predefined threshold. For each of the monitored spatial positionings a particular predefined threshold can be specified, in particular via a user input. When the computed difference between the previously and currently detected spatial positioning of the first and/or the second display device exceeds the predefined threshold, the execution of steps e) to f) can be repeated.

Advantageously, the relative spatial positioning between the first and second display device can be monitored by the second tracking element. If the spatial positionings of the first and second display device are equally changing, in particular if the relative spatial positioning between the first and second display device remains unchanged, a repeated execution of steps e) to f) may not become necessary. When a change in the relative spatial positioning between the first and second display device is detected by the second tracking element, steps e) to f) can be repeated. Thereby the graphical representation of the augmented reality can be adapted such that it immerses into the graphical representation of the image data displayed by the first display device.

When steps e) to f) are repeated, at least part of the graphical representation of the augmented reality can be adapted such that it spatially and/or visually matches with the graphical representation of the image data, in particular an anatomical and/or geometrical feature. The adaption of the at least part of the graphical representation of the augmented reality can comprise a spatial and/or visual matching of the graphical representation of the augmented reality. The spatial and/or visual matching of the at least part of the graphical representation of the augmented reality can comprise a rotation and/or translation and/or scaling and/or deformation and/or truncation of the at least part of the graphical representation of the augmented reality based on the detected change in the relative spatial positioning between the first and second display device.

Advantageously, the aforementioned embodiment permits an immersive display of the augmented reality and the image data. In particular, when the second display device is embodied as a head-mounted display device, steps e) to f) can be repeated whenever a motion of the user's head induces a change in the relative spatial positioning between the first and second display device, in particular beyond the predefined threshold.

In a third aspect, the current invention concerns a computer program product. The computer program product can comprise a computer program. The computer program according to the invention may, for example, be directly loaded into a memory of a processing unit, in particular a control device of a medical imaging system, and comprises program means to perform the steps of a method according to the invention if the computer program is executed in the processing unit. The computer program may be stored on an electronically readably storage medium, which thus comprises electronically readable control information stored thereon. The control information comprises at least a computer program according to the invention and is configured such that the control information executes a method according to the invention when the storage medium is used in a processing unit, in particular a control device of a medical imaging system. The electronically readably storage medium according to the invention may preferably be a non-transient medium, in particular a CD-ROM. The computer program product may comprise further elements, such as a documentation and/or additional components, in particular hardware dongles for using the software.

In addition, the current invention may also emanate from an electronically readably storage medium, which stores electronically readable control information such that the control information executes a method according to the invention when the storage medium is used in a processing unit.

A largely software-based implementation bears the advantage that previously used processing units can be easily upgraded via a software update in order to execute a method according to the present invention.

Further details and advantages of the current invention become apparent from the following detailed description of preferred embodiments taken in conjunction with the drawings, in which:
Figs. 1 to 3 show schematic representations of different exemplary embodiments of a proposed system for displaying an augmented reality,
Figs. 4 to 7 show schematic representations of different exemplary embodiments of a proposed method for generating an augmented reality.

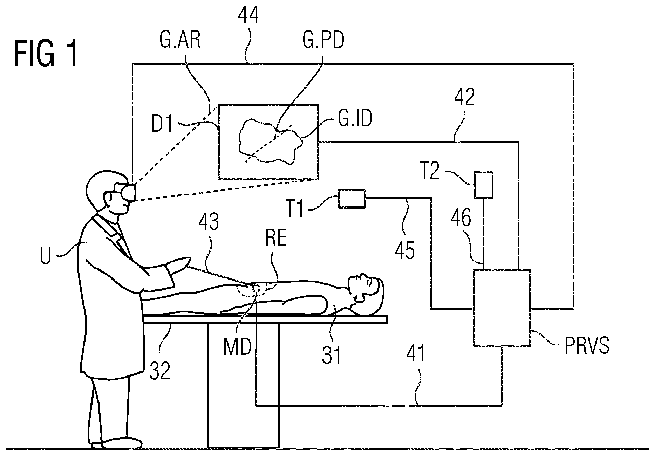

Fig. 1 depicts a schematic representation of an exemplary embodiment of a proposed system for displaying an augmented reality. The system can comprise a first display device D1, a second display device D2, a first tracking element T1, a second tracking element T2 and a medical device MD. Further, the medical device MD can be configured to acquire image data of a region under examination RE of a subject 31, wherein the subject 31 can be positioned on a patient support 32. The medical device MD can comprise a diagnostic and/or surgical device which can be configured to acquire, in particular intra-procedural and/or real-time, image data of the region under examination RE of the subject 31. In particular, the medical device MD can comprise a catheter and/or endoscope and/or laparoscope, which can be configured to be at least partially placed inside the region under examination RE of the subject 31. The medical device MD can at least in parts be flexible and/or rigid. The medical device MD can be configured to intra-operatively acquire the image data from the region under examination RE. Further, the medical device MD can comprise an angled lens for acquiring the image data. The region under examination RE of the subject 31 may comprise an anatomical region and/or a hollow organ and/or body cavity of the subject 31. Advantageously, the image data can comprise a 2D and/or 3D representation of at least part of the region under examination RE. In addition, the image data can be time-resolved. Moreover, the medical device MD can be configured to provide the image data to the first display device D1. For this purpose, the medical device MD can communicate with a processing unit PRVS via a signal 41. Moreover, the processing unit PRVS can be configured to provide the image data to the first display device D1 via a signal 42.

The first display device D1 can be configured to display a graphical representation of the image data G.ID. In particular, the first display device D1 can comprise a monitor and/or display, which is configured to display the graphical representation of the image data G.ID, in particular in real-time. Advantageously, the first display device D1 can comprise a high-resolution display, in particular a 2K-display and/or a 4K-display and/or an 8K-display.

Moreover, the first tracking element T1 can be configured to detect a spatial positioning of the medical device MD. The first tracking element T1 can comprise a sensor, exemplary an electromagnetic and/or optical and/or ultrasound sensor, which is configured to detect a spatial positioning of the medical device MD. Further, the first tracking element T1 can be configured to detect and/or track and/or monitor the spatial positioning, in particular a spatial position and/or orientation, of the medical device MD, in particular in a coordinate frame of the subject 31 and/or a reference coordinate frame. Furthermore, the medical device MD can comprise a first marker element (not shown), which can be identified and spatially located by the first tracking element T1. The first marker element can be embodied as a predefined shape and/or contour and/or segment of the medical device MD, in particular a fiducial marker. Furthermore, the first marker element can be embodied as at least one marker object which can be attached to the medical device MD, in particular on a distal and/or proximal portion of the medical device MD. Exemplary, the first tracking element T1 can comprise a camera, in particular a mono and/or stereo camera, which can be configured to detect and/or track and/or monitor the first marker element. Advantageously, the first tracking element T1 can be configured to provide the detected spatial positioning of the medical device MD to the processing unit PRVS via a signal 45.

Furthermore, the second tracking element T2 can be configured to detect the spatial positionings of the first display device D1 and the second display device D2. Advantageously, the second tracking element T2 can comprise a sensor, exemplary an electromagnetic and/or optical and/or ultrasound sensor, which is configured to, in particular simultaneously, detect a spatial positioning of the first D1 and second display device D2. Further, the second tracking element T2 can be configured to detect and/or track and/or monitor the spatial positioning, in particular a spatial position and/or orientation, of the first D1 and second display device D2, in particular relative to each other and/or in a common coordinate frame. The second tracking element T2 can be configured to provide the detected spatial positionings of the first D1 and second display device D2 to the processing unit PRVS via a signal 46.

The first display device D1 can comprise a second marker element (not shown) and/or the second display device D2 can comprise a third marker element (not shown), wherein the second tracking element T2 can be configured to identify and spatially locate the second and/or the third marker element. The second marker element can be embodied as a predefined shape and/or contour and/or segment of the first display device D1, in particular a fiducial marker. Furthermore, the second marker element can be embodied as at least one marker object which can be attached to the first display device D1. Analogously, the third marker element can be embodied as a predefined shape and/or contour and/or segment of the second display device D2, in particular a fiducial marker. Moreover, the third marker element can be embodied as at least one marker object which can be attached to the second display device D2.

Advantageously, the second tracking element T2 can be configured to detect a relative and/or absolute spatial positioning between the first and second display device. Furthermore, the second tracking element T2 can comprise a camera, in particular a mono and/or stereo camera, which is configured to detect and/or track and/or monitor the second and/or the third marker element.

The system, in particular the processing unit PRVS, can further be configured to receive a planning dataset. The planning dataset can comprise a, in particular pre-procedural, 2D and/or 3D representation of at least part of the region under examination RE of the subject 31. In addition, the planning dataset can be time-resolved. The planning dataset may further comprise pre-processed information, for example annotations and/or anatomical landmarks and/or geometrical landmarks and/or enhanced features. In addition, the planning dataset can comprise planning information regarding a path for the medical device MD and/or information regarding a treatment plan.

Furthermore, the system can be configured to segment at least part of the planning dataset and to generate the augmented reality based on the segmented planning dataset.

In addition, the system can be configured to generate the augmented reality based on the planning dataset and the detected spatial positioning of the medical device MD. Further, the planning dataset can be spatially arranged with respect to the first display device D1 in accordance with the detected spatial positionings of the first D1 and second display device D2. In addition, the second display device D2 can be configured to display a graphical representation of the augmented reality G.AR.

The second display device D2 can comprise a second display interface, which can be configured to receive the augmented reality, in particular via a signal 44 from the processing unit PRVS. In particular, the second display device D2 can be configured as a pair of goggles, in particular data goggles, and/or a helmet, in particular a data helmet, and/or a screen and/or projector, which is wearable by the user U. When the second display device D2 is worn by the user U, in particular at least partially on the user's head, the second display device D2 will move uniformly with the user U, in particular the user's head. Hereby, the user U can perceive the graphical representation of the augmented reality G.AR and simultaneously control and/or actuate and/or position the medical device MD, in particular via a control interface 43. The control interface 43 can be located at a distal portion of the medical device MD.

Furthermore, the second tracking element T2 can be arranged spatially apart from the first D1 and second display device D2. Further, the first T1 and second tracking element T2 can be embodied as a single sensor (not shown).

In addition, the system can be configured to monitor at least one of the detected spatial positionings and adapt the augmented reality when a change in the at least one of the detected spatial positionings occurs.

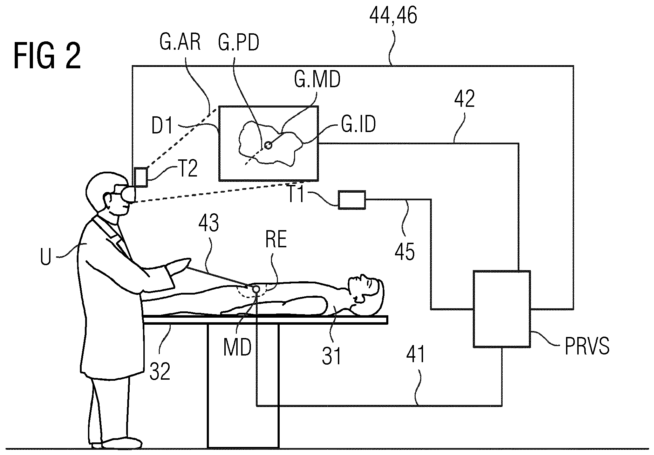

Fig. 2 shows another exemplary embodiment of a proposed system for displaying an augmented reality, wherein the system can be configured to generate the augmented reality comprising a virtual representation of the medical device G.MD based on the detected spatial positioning of the medical device MD. Advantageously, the graphical representation of the augmented reality G.AR can comprise depth cue information with regard to the planning dataset and/or the virtual representation of the medical device G.MD.

Furthermore, the second tracking element T2 can be mounted, in particular attached, to the second display device D2 in a predefined position, in particular a fixed position with respect to the second display device D2. Advantageously, the second tracking element T2 can be mounted to the second display device D2 such that a spatial detection range of the second tracking element T2 at least partially coincides with a field-of-view of the user U. Thereby, it can be ensured, that the relative spatial positioning between the first D1 and second display device D2 can be detected by the second tracking element T2 whenever the user U turns its field-of-view towards the first display device D1.

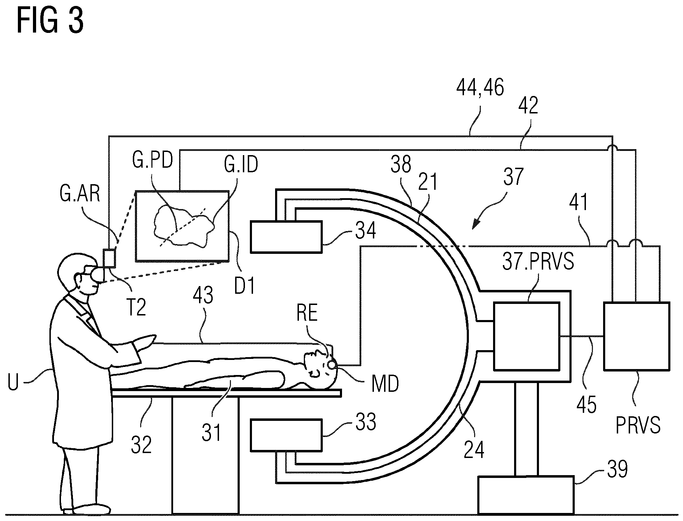

Fig. 3 shows another exemplary embodiment of a proposed system for displaying an augmented reality, wherein the first tracking element T1 can be embodied as a medical imaging system, in particular a medical C-arm X-ray system 37.

The medical C-arm X-ray system 37 can be configured to, in particular recurrently and/or continuously, acquire further medical image data of the region under examination RE and to detect the spatial positioning of the medical device MD based on the further medical image data.

When the first tracking element T1 is embodied as a medical C-arm X-ray system 37 and the planning dataset was acquired by that medical C-arm X-ray system 37, the detected spatial positioning of the medical device MD can inherently be registered to the planning dataset. The medical C-arm X-ray system 37 can comprise a further processing unit 37.PRVS, which can be in communication with the processing unit PRVS via the signal 45. The medical C-arm X-ray system 37 can further comprise an X-ray detector unit 34 and an X-ray source 33, which can be mounted to the C-arm 38 of the C-arm X-ray system 37 such that they are movable around at least one axis. In addition, the medical C-arm X-ray system 37 can comprise a motion unit 39, in particular comprising at least a wheel and/or rail and/or robotic system, which permits a spatial motion of the medical C-arm X-ray system 37.

For the acquisition of the further medical image data and/or the planning dataset, in particular comprising at least one projection image of the region under examination RE of the subject 31, the further processing unit 37.PRVS can send a signal 24 to the X-ray source 33. Consequently, the X-ray source 33 can emit an X-ray bundle, in particular a cone-beam and/or a fan-beam and/or a parallel-beam. When the X-ray bundle impinges on a surface of the X-ray detector unit 34 after an interaction between the X-ray bundle and the region under examination RE of the subject 31, the X-ray detector unit 34 can send a signal 21 to the further processing unit 37.PRVS. Based on the signal 21 the further processing unit 37.PRVS can be configured to receive the further medical image data and/or the planning dataset. Moreover, the further processing unit 37.PRVS can provide the further medical image data and/or the planning dataset and/or the spatial positioning of the medical device MD to the processing unit PRVS via the signal 45.

Furthermore, the medical C-arm X-ray system 37 can be configured to acquire a device dataset, wherein the system, in particular the processing unit PRVS and/or the further processing unit 37.PRVS, can be configured to segment the medical device MD in the device dataset and to generate the virtual representation of the medical device G.MD based on the segmented device dataset.

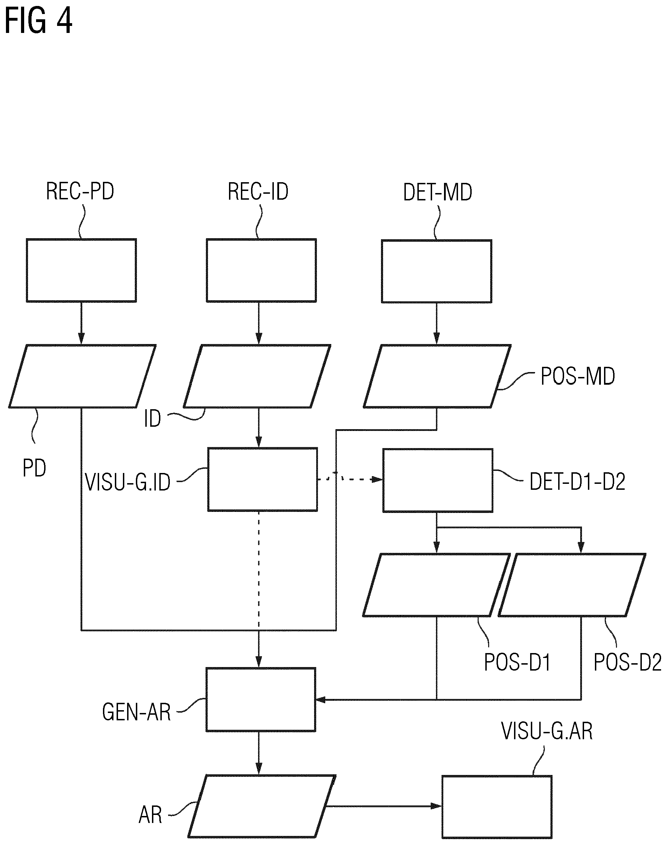

Fig. 4 depicts a schematic representation of an exemplary embodiments of a proposed method for generating an augmented reality. In a first step a), the planning dataset PD can be received REC-PD. In a second step b), the image data ID of the region under examination RE of the subject 31 can be received REC-ID from the medical device MD. In a third step c), the graphical representation of the image data G.ID can be displayed VISU-G.ID with the first display device D1. In a fourth step d), the spatial positioning of the medical device POS-MD can be detected DET-MD with the first tracking element T1. In a fifth step e), the spatial positionings of the first display device POS-D1 and the second display device POS-D2 can be detected DET-D1-D2 with the second tracking element T2. In a sixth step f), the augmented reality AR can be generated GEN-AR based on the planning dataset PD and the detected spatial positioning of the medical device POS-MD.

Furthermore, the planning dataset PD can be spatially arranged with respect to the first display device D1 in accordance with the detected spatial positionings of the first POS-D1 and second display device POS-D2. In a seventh step g), a graphical representation of the augmented reality G-AR can be displayed VISU-G.AR with the second display device D2.

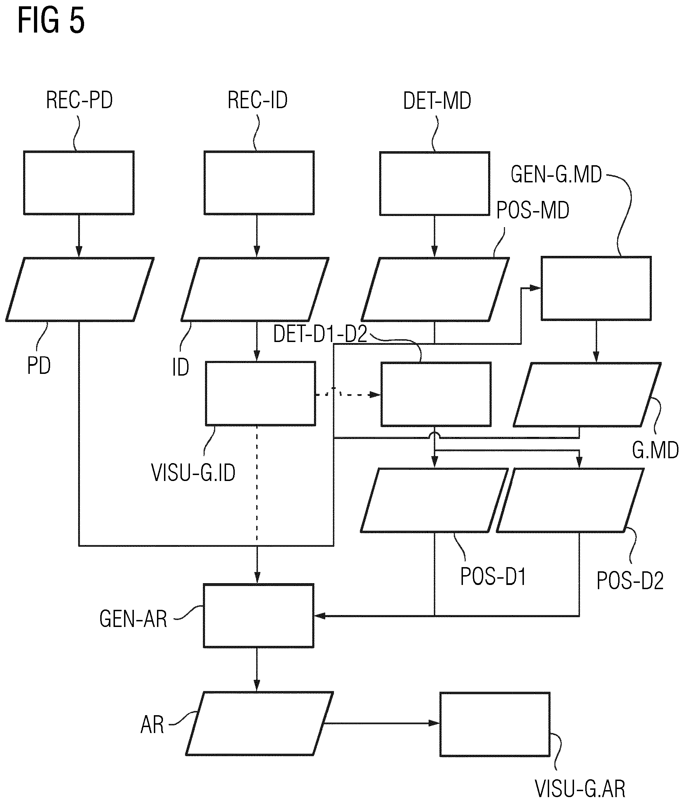

Fig. 5 shows another exemplary embodiment of the proposed method for generating an augmented reality, wherein the augmented reality AR can be generated GEN-AR comprising a virtual representation of the medical device G.MD based on the detected spatial positioning of the medical device POS-MD.

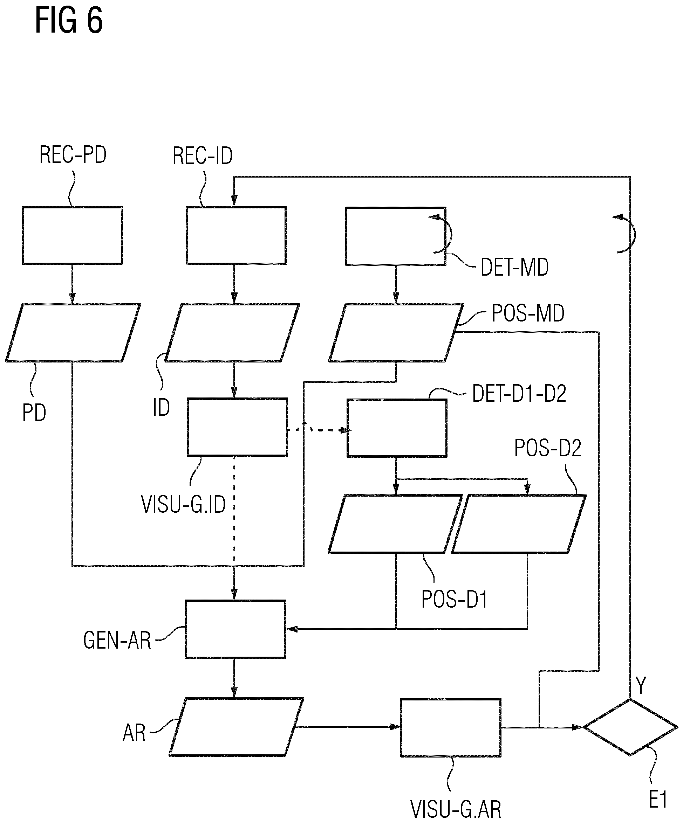

Fig. 6 shows another exemplary embodiment of the proposed method for generating an augmented reality, wherein the execution of steps b) to f) can be repeated when a change in the spatial positioning of the medical device POS-MD is detected DET-MD. A change in the spatial positioning of the medical device POS-MD can advantageously be detected DET-MD by the first tracking element T1. In particular, the first tracking element T1 can monitor the spatial positioning of the medical device POS-MD by either continuously or recurrently detecting the spatial positioning POS-MD, in particular after a predefined time interval. Further, a difference between the previously and currently detected spatial positioning of the medical device POS-MD can be computed and compared E1 to a predefined threshold. When the computed difference between the previously and currently detected spatial positioning of the medical device POS-MD exceeds Y the predefined threshold, the execution of steps b) to f) can be repeated.

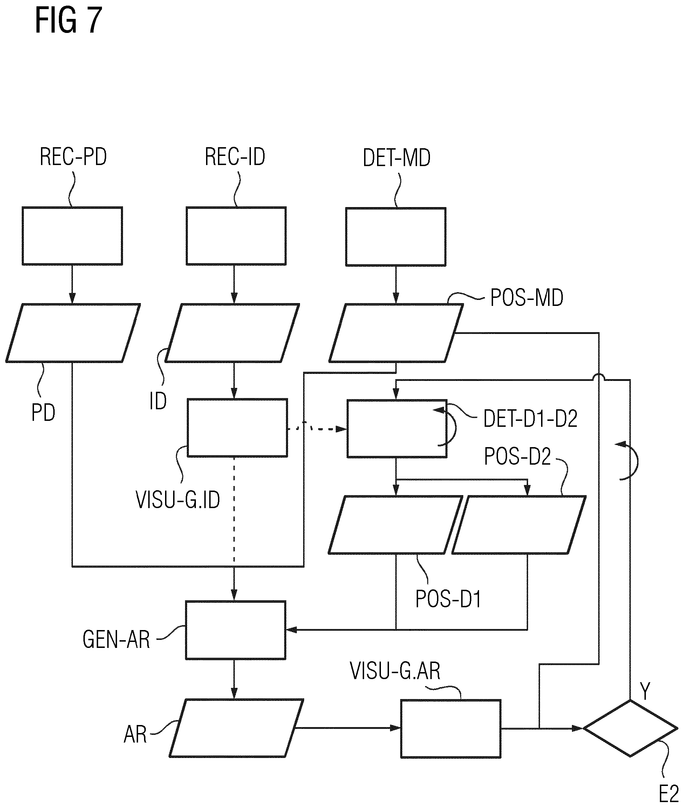

Fig. 7 shows another exemplary embodiment of the proposed method for generating an augmented reality, wherein the execution of steps e) to f) can be repeated when a change in the spatial positioning of the first display device POS-D1 and/or the spatial positioning of the second display device POS-D2 is detected. A change in the spatial positioning of the first POS-D1 and/or the second display device POS-D2 can advantageously be detected DET-D1-D2 by the second tracking element T2. In particular, the second tracking element T2 can monitor the spatial positionings of the first POS-D1 and the second display device POS-D2 by either continuously or recurrently detecting the spatial positionings DET-D1-D2, in particular after a predefined time interval. Further, a difference between the previously and currently detected spatial positionings of the first POS-D1 and/or the second display device POS-D2 can be computed and compared E2 to a predefined threshold. When the computed difference between the previously and currently detected spatial positionings of the first POS-D1 and/or the second display device POS-D2 exceeds Y the predefined threshold, the execution of steps e) to f) can be repeated.

Although the present invention has been described in detail with reference to preferred embodiments, the present invention is not limited by the disclosed examples from which the skilled person is able to derive other variations without departing from the scope of the invention. In addition, the utilization of indefinite articles such as "a" and/or "an" does not exclude multiples of the respective features. Moreover, terms such as "unit" and "element" do not exclude that the respective components may comprise multiple interacting sub-components, wherein the sub-components may further be spatially distributed.

## Claims

1. System for displaying an augmented reality (VISU-AR), the system comprising a first (D1) and a second display device (D2), a first (T1) and a second tracking element (T2) and a medical device (MD),
wherein the medical device (MD) is configured to:
- acquire image data (ID) of a region under examination (RE) of a subject (31),
- provide the image data (ID) to the first display device (D1),
wherein the first display device (D1) is configured to display a graphical representation of the image data (VISU-G.ID),
wherein the first tracking element (T1) is configured to detect a spatial positioning of the medical device (DET-MD),
wherein the second tracking element (T2) is configured to detect spatial positionings of the first display device and the second display device (DET-D1-D2),
wherein the system is configured to:
- receive a planning dataset (REC-PD),
- generate the augmented reality (GEN-AR) based on the planning dataset (PD) and the detected spatial positioning of the medical device (POS-MD),
wherein the planning dataset (PD) is spatially arranged with respect to the first display device (D1) in accordance with the detected spatial positionings of the first (POS-D1) and second display device (POS-D2),
wherein the second display device (D2) is configured to display a graphical representation of the augmented reality (VISU-G.AR).

2. System in accordance with claim 1, **characterized in that** the system is further configured to generate the augmented reality (GEN-AR) comprising a virtual representation of the medical device (G.MD) based on the detected spatial positioning of the medical device (POS-MD).

3. System in accordance with claim 1 or 2, **characterized in that** the system is further configured to:
- monitor at least one of the detected spatial positionings,
- adapt the augmented reality when a change in the at least one of the detected spatial positionings occurs.

4. System in accordance with any of the preceding claims, **characterized in that** the second tracking element (T2) is mounted to the second display device (D2) in a predefined position.

5. System in accordance with any of claims 1 to 3, **characterized in that** the second tracking element (T2) is arranged spatially apart from the first (D1) and second display device (D2).

6. System in accordance with any of the preceding claims, **characterized in that** the system is further configured to:
- segment at least part of the planning dataset (PD),
- generate the augmented reality (GEN-AR) based on the segmented planning dataset.

7. System in accordance with any of the preceding claims, wherein the first tracking element (T1) is embodied as a medical imaging system.

8. System in accordance with claims 2 and 7, wherein the medical imaging system is configured to acquire a device dataset,
wherein the system is further configured to:
- segment the medical device (MD) in the device dataset,
- generate the virtual representation of the medical device (G.MD) based on the segmented device dataset.

9. System in accordance with any of the preceding claims, **characterized in that** the second display device (D2) is at least partially embodied as a head-mounted display device which is wearable by a user (U).

10. System in accordance with any of the preceding claims,
wherein the medical device (MD) is embodied as an endoscope and/or a catheter and/or a laparoscope,
wherein the medical device (MD) comprises an angled lens for acquiring the image data (ID).

11. Method for generating an augmented reality (GEN-AR), comprising:
a) receive a planning dataset (REC-PD),
b) receive image data (REC-ID) of a region under examination (RE) of a subject (31) from a medical device (MD),
c) display (VISU-G.ID) a graphical representation of the image data (G.ID) with a first display device (D1),
d) detect (DET-MD) a spatial positioning of the medical device (POS-MD) with a first tracking element (T1),
e) detect (DET-D1-D2) spatial positionings of the first display device (POS-D1) and the second display device (POS-D2) with a second tracking element (T2),
f) generate the augmented reality (GEN-AR) based on the planning dataset (PD) and the detected spatial positioning of the medical device (POS-MD),
wherein the planning dataset (PD) is spatially arranged with respect to the first display device (D1) in accordance with the detected spatial positionings of the first (POS-D1) and second display device (POS-D2),
g) display a graphical representation of the augmented reality (VISU-G.AR) with the second display device (D2).

12. Method in accordance with claim 11, wherein the augmented reality (AR) is generated comprising a virtual representation of the medical device (G.MD) based on the detected spatial positioning of the medical device (POS-MD).

13. Method in accordance with claim 11 or 12, wherein execution of the steps b) to f) is repeated when a change in the spatial positioning of the medical device (POS-MD) is detected (DET-MD).

14. Method in accordance with any of claims 11 to 13, wherein execution of the steps e) to f) is repeated when a change in the spatial positioning of the first (POS-D1) and/or the second display device (POS-D2) is detected (DET-D1-D2).

15. Computer program product, which comprises a program and is directly loadable into a memory of a programmable controller of a processing unit (PRVS), with program code sections, to execute all steps of a method according to one of the claims 11 to 14 when the program is executed in the programmable controller of the processing unit (PRVS).
